# EUROPEAN PATENT APPLICATION

(11) **EP 4 620 379 A1**
(43) Date of publication of application: **24.09.2025**
(21) Application number: 25164628.7
(22) Date of filing: 19.03.2025
(51) Int. Cl.: A61B 3/00, A61B 3/032, A61B 3/08, A61B 3/13

(54) **OPHTHALMOLOGIC APPARATUS**

(30) Priority: 22.03.2024 JP 2024047093
(71) Applicant: TOPCON CORPORATION, Tokyo 174-8580 (JP)
(72) Inventor: YUKIMORI, Takafumi, Tokyo 174-8580 (JP); TATARA, Yoko, Tokyo 174-8580 (JP)
(74) Representative: Louis Pöhlau Lohrentz

(57) **Abstract**

An ophthalmologic apparatus (1) includes a visual target presenting mechanism (44) that is configured to present a left-eye visual target (OtL) to a left subject eye (EL) and a right-eye visual target (OtR) to a right subject eye (ER). The left-eye and right-eye visual targets (OtL, OtR) have corresponding configurations and respectively include a corresponding portion (s1L, s1R) and a remaining portion (s2L, s2R). The visual target presenting mechanism (44) is configured to increase a brightness difference between the corresponding portions (s1L, s1R) and maintain a brightness difference between the remaining portions (s2L, s2R).

## Description

### FIELD OF THE INVENTION

The present invention relates to an ophthalmologic apparatus.

### BACKGROUND

JP2017-169601A1 discloses an ophthalmologic apparatus that estimates the eyestrain level of the subject eyes based on the timing at which binocular fusion is disrupted (also referred to as binocular fusion disruption hereinafter) when the vision of one subject eye is gradually darkened from the state in which visual targets are simultaneously viewed by both subject eyes.

The conventional ophthalmologic apparatus determines the timing of the binocular fusion disruption by detecting the occurrence of the ocular position displacement (visual line misalignment) of one subject eye. However, if the conventional ophthalmologic apparatus fails to detect the ocular position displacement, it cannot determine the timing of the binocular fusion disruption, resulting in the inability to properly estimate the eyestrain level of the subject eyes.

The present invention has been made in view of the above problem. An object of the present invention aims to provide an ophthalmologic apparatus capable of easily detecting ocular position displacement when the brightness difference between the visual targets presented to the left and right subject eyes is increased from the binocular vision condition.

### SUMMARY

In one embodiment according to the present invention, an ophthalmologic apparatus includes a visual target presenting mechanism that is configured to present a left-eye visual target to a left subject eye and a right-eye visual target to a right subject eye. The left-eye and right-eye visual targets have corresponding configurations and respectively include a corresponding portion and a remaining portion. The visual target presenting mechanism is configured to increase a brightness difference between the corresponding portions and maintain a brightness difference between the remaining portions.

The ophthalmologic apparatus according to the present invention enables easier detection of the ocular position displacement when the brightness difference between the visual targets presented to the left and right subject eyes is increased from the binocular vision condition.

### BRIEF DESCRIPTION OF DRAWINGS

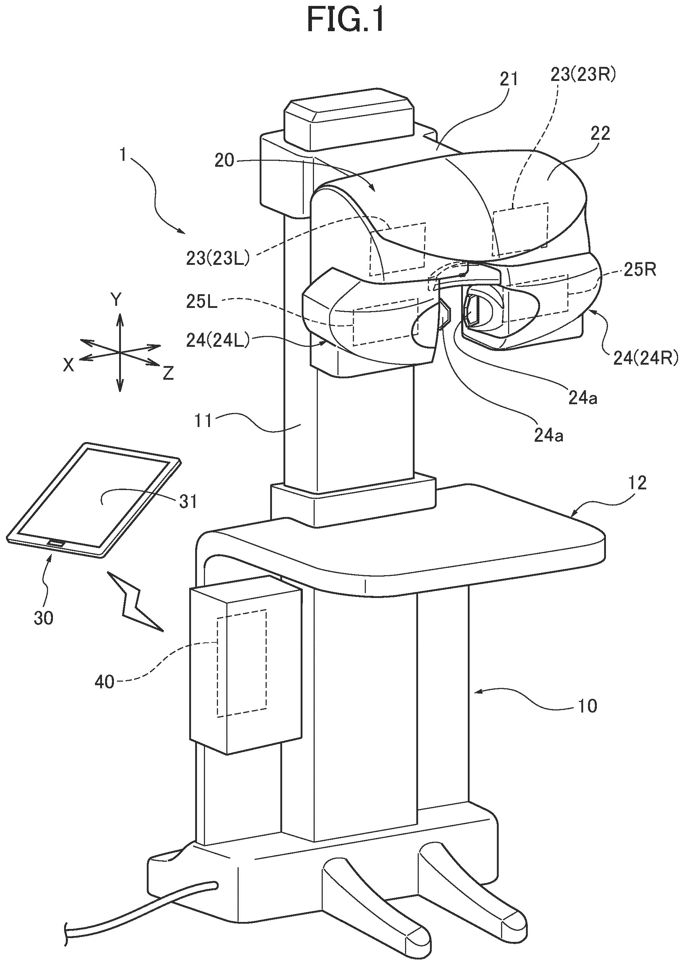

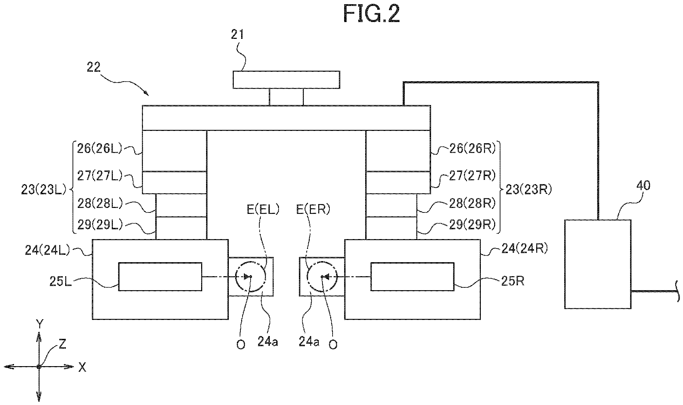

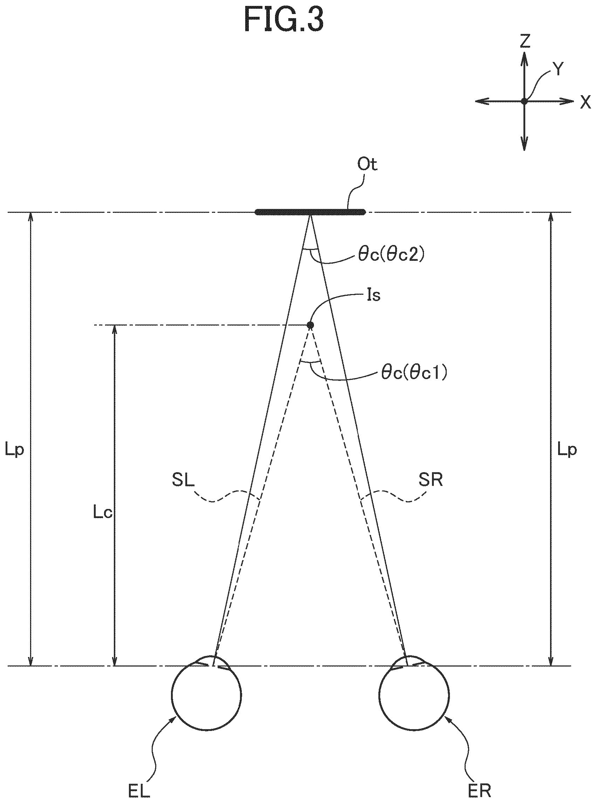

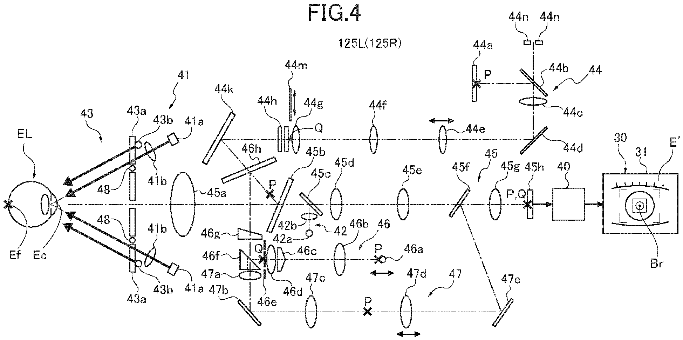

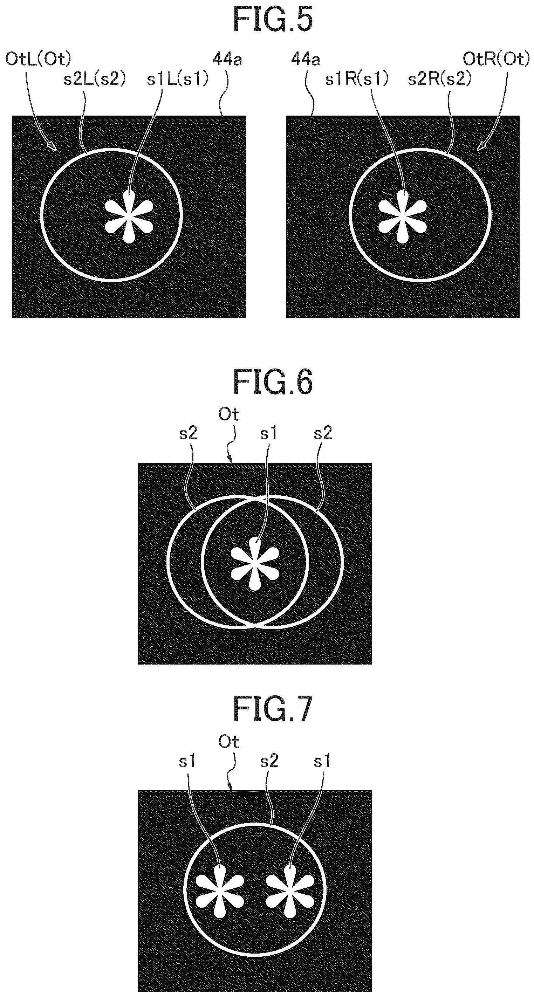

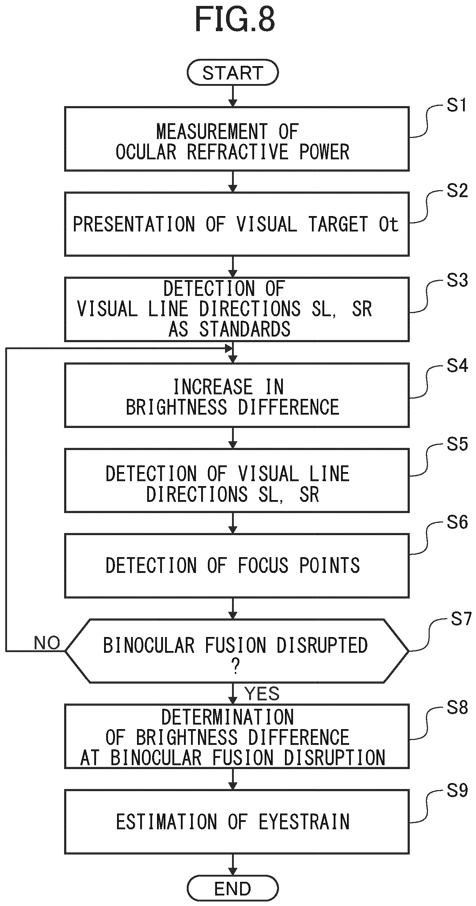

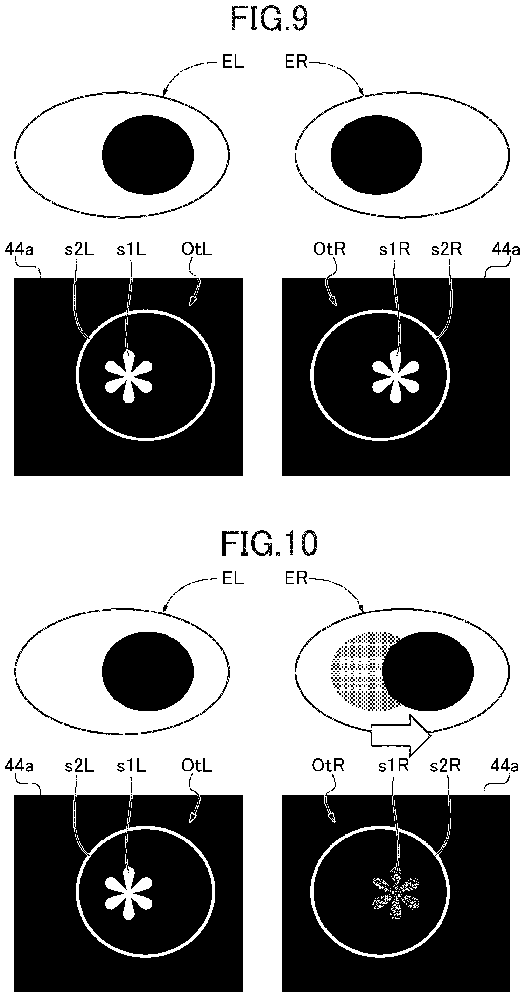

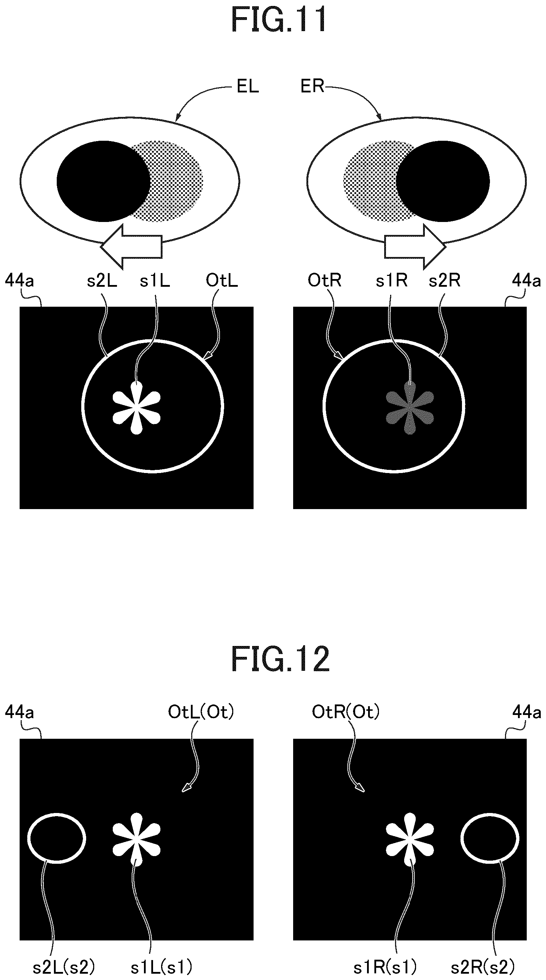

FIG. 1 is an external perspective view illustrating an ophthalmologic apparatus according to a first embodiment. FIG. 2 is an explanatory view schematically illustrating a drive mechanism of a measurement head of the ophthalmologic apparatus according to the first embodiment. FIG. 3 is an explanatory view illustrating a convergence angle and a visual target presentation distance. FIG. 4 is an explanatory view illustrating the configuration of a left measurement optical system of the ophthalmologic apparatus according to the first embodiment. FIG. 5 is an explanatory view illustrating left-eye and right-eye visual targets according to the first embodiment. FIG. 6 is an explanatory view illustrating a condition of the binocular fusion of first visual target signs in FIG. 5. FIG. 7 is an explanatory view illustrating a condition of the binocular fusion of second visual target signs in FIG. 5. FIG. 8 is a flowchart illustrating a flow of an eyestrain estimation process by a controller according to the first embodiment. FIG. 9 is an explanatory view schematically illustrating, at the top, the left and right subject eyes in a front view, and at the bottom, the corresponding left-eye and right-eye visual targets. FIG. 10 is an explanatory view illustrating, from FIG. 9, a condition where the brightness of the first visual target sign for the left-eye visual target is reduced, and the gaze of the left subject eye shifts from the first visual target sign to the second visual target sign. FIG. 11 is an explanatory view illustrating a condition in which, as the gaze of the left subject eye is directed toward the second visual target sign, the gaze of the right subject eye shifts from the first visual target sign to the second visual target sign. FIG. 12 is an explanatory view showing the left-eye and right-eye visual targets in another example.

### DETAILED DESCRIPTION

Regarding the use of plural and singular terms herein, those skilled in the art can interpret plural terms as singular and singular terms as plural, as necessary for the context or application. Variations in singular and plural usage may be explicitly stated herein for clarity.

An embodiment of an ophthalmologic apparatus according to the present invention will be described with reference to FIGS. 1 to 12.

### First Embodiment

An ophthalmologic apparatus 1 according to the first embodiment includes an optical system for measuring the eye characteristics of the subject eyes and is configured to objectively and subjectively measure the eye characteristics of the subject eyes. The examiner can use the ophthalmologic apparatus 1 to perform objective and subjective examinations. In the objective examination, the ophthalmologic apparatus 1 projects light onto the subject eyes and measures (obtains) the ocular information (eye characteristics) based on the detection results of the reflected light. The objective examinations include measurements to obtain characteristics of the subject eyes as the ocular information and imaging to capture images of the subject eyes. Additionally, the objective examinations include the objective refractive measurement (refraction measurement), the corneal shape measurement (keratometry measurement), the intraocular pressure measurement, the ocular fundus imaging, the tomogram imaging using optical coherence tomography (hereinafter referred to as OCT) (i.e., OCT imaging), and other measurements using OCT. In the subjective examination, the ophthalmologic apparatus 1 presents a visual target to the examinee and measures information about the subject eyes (eye characteristics) based on the responses from the examinee to the visual target. The subjective examination includes subjective refraction tests such as the far-point test, the intermediate-point test, the near-point test, the contrast test, the glare test, the RG test, and the visual field test.

As shown in FIG. 1, the ophthalmologic apparatus 1 according to the first embodiment is a both-eye open type apparatus capable of simultaneously measuring the eye characteristics of both eyes at the same time with an examinee opening both eyes. The ophthalmologic apparatus 1 may also individually measure the eye characteristics of each eye at a time by shielding one of the eyes or turning off a fixed target for one of the eyes.

The ophthalmologic apparatus 1 includes a support base 10, a measurement unit 20, an examiner's control device 30, a controller 40, and an examinee's control device (not shown). From the perspective of the examinee facing the ophthalmologic apparatus 1, the horizontal axis in the left and right direction (i.e., horizontal direction) is indicated with an arrow X (X-axis), the vertical axis in the up-down direction (i.e., vertical direction) is indicated with an arrow Y (Y-axis), and the depth axis in the front-back direction (i.e., depth direction), orthogonal to both the horizontal and vertical directions, is indicated wit arrow Z (Z-axis).

The support base 10 includes a column 11 rising from a floor and an optometric table 12 supported by the column 11. The optometric table 12 is a base for supporting a posture of the examinee. The optometric table 12 is also used to place a device and a tool for use in optometry, such as the examiner's control device 30, thereon. The optometric table 12 may be supported on the column 11 to adjust the position (height position) of the optometric table 12 in the Y direction.

The measurement unit 20 includes an arm 21 and a measurement head 22. One end of the arm 21 is supported by an end portion of the column 11, and the other end of the arm 21 extends toward a near side (i.e., the examinee's side) from the column 11 in the Z direction. The measurement head 22 is attached to an end of the arm 21. The measurement head 22 is thereby hung on the column 11 via the arm 21 above the optometric table 12. The arm 21 is configured to move in the Y direction relative to the column 11. The arm 21 may be configured to move in the X and Z directions relative to the column 11.

The measurement head 22 is configured to individually measure the eye characteristics of the left and right subject eyes EL, ER. The measurement head 22 includes a left-eye drive mechanism (i.e., a drive mechanism for the left eye) 23L and a right-eye drive mechanism (i.e., a drive mechanism for the right eye) 23R, which are attached to the distal end of the arm 21. The measurement head 22 also includes a left measurement portion 24L and a right measurement portion 24R, which are provided below the left-eye drive mechanism 23L and the right-eye drive mechanism 23R, respectively.

The left and right measurement portions 24L, 24R are provided as a pair, respectively corresponding to the left and right subject eyes EL, ER of the examinee. The left measurement portion 24L includes a left measurement optical system 25L for measuring the eye characteristics of the left subject eye EL. The right measurement portion 24R includes a right measurement optical system 25R for measuring the eye characteristics of the right subject eye ER. The left and right measurement portions 24L, 24R are configured to provide the measurement results to the controller 40.

The left-eye drive mechanism 23L is configured to move the left measurement portion 24L horizontally (in the X direction) and vertically (in the Y direction) and to rotate it about the X and Y axes. As shown in FIG. 2, the left-eye drive mechanism 23L includes a left vertical drive portion 26L, a left horizontal drive portion 27L, a left Y-axis rotation drive portion 28L, and a left X-axis rotation drive portion 29L. The right-eye drive mechanism 23R is configured to move the right measurement portion 24R horizontally (in the X direction) and vertically (in the Y direction) and to rotate it about the X and Y axes. The right-eye drive mechanism 23R includes a right vertical drive portion 26R, a right horizontal drive portion 27R, a right Y-axis rotation drive portion 28R, and a right X-axis rotation drive portion 29R.

The left-eye and right-eye drive mechanisms 23L, 23R are symmetrically arranged relative to a vertical plane located midway between them in the X direction. Hereinafter, unless otherwise individually specified, the left-eye and right-eye drive mechanisms 23L, 23R are collectively referred to as the drive mechanism(s) 23, the left and right measurement portions 24L, 24R as the measurement portion(s) 24, the left and right vertical drive portions 26L, 26R as the vertical drive portion(s) 26, the left and right horizontal drive portions 27L, 27R as the horizontal drive portion(s) 27, the left and right Y-axis rotation drive portions 28L, 28R as the Y-axis rotation drive portion(s) 28, and the left and right X-axis rotation drive portions 29L, 29R as the X-axis rotation drive portion(s) 29.

The vertical drive portions 26 are provided between the arm 21 and the horizontal drive portions 27 and are configured to move the horizontal drive portions 27 in the Y direction (vertical direction) relative to the arm 21. The horizontal drive portions 27 are provided between the vertical drive portions 26 and the Y-axis rotation drive portions 28 and are configured to move the Y-axis rotation drive portions 28 in the X and Z directions (horizontal direction) relative to the vertical drive portions 26. The vertical drive portions 26 and the horizontal drive portions 27 each include an actuator, such as a pulse motor, to generate a driving force, and a transmission mechanism, such as a combination of gears or a rack and pinion, to transmit the driving force. Each of the horizontal drive portions 27 is designed to facilitate easy configuration and control of the movement in the horizontal direction, for example, by separately incorporating a combination of actuators and transmission mechanisms for the X and Z directions, respectively.

The Y-axis rotation drive portions 28 are provided between the horizontal drive portions 27 and the X-axis rotation drive portions 29 and are configured to rotate the X-axis rotation drive portions 29 relative to the horizontal drive portions 27 about respective eyeball rotation Y axes. The eyeball rotation Y axes extend in the Y direction through the eyeball rotation points O of the corresponding subject eyes E. The X-axis rotation drive portions 29 are provided between each of the Y-axis rotation drive portions 28 and their corresponding measurement portions 24. The X-axis rotation drive portions 29 are configured to rotate each of the measurement portions 24 about respective eyeball rotation X axes, relative to the Y-axis rotation drive portions 28. The eyeball rotation X axes extend in the X direction through the eyeball rotation points O of the corresponding subject eyes E.

Each of the Y-axis rotation drive portions 28 and the X-axis rotation drive portions 29 includes, for example, an actuator and a transmission mechanism, similar to the vertical drive portions 26 and the horizontal drive portions 27 and is configured such that the transmission mechanism receiving the driving force from the actuator moves along an arc-shaped guide groove. The central position of the guide groove is aligned with the eyeball rotation Y axis and thus, the Y-axis rotation drive portion 28 can rotate the measurement portion 24 about the eyeball rotation Y axis of the corresponding subject eye E. The central position of the guide groove is aligned with the eyeball rotation X axis and thus, the X-axis rotation drive portion 29 can rotate the measurement portion 24 about the eyeball rotation X axis of the corresponding subject eye E. **In** other words, the central position of each guide groove of the Y-axis rotation drive portions 28 and the X-axis rotation drive portions 29 is aligned with the eyeball rotation point O of the corresponding subject eye E, and thus, the measurement portions 24 can rotate about eyeball rotation points O in the left and right direction (rotation direction about the Y direction) and the vertical direction (rotation direction about the X direction).

The Y-axis rotation drive portions 28 may be configured to rotatably support the measurement portions 24 about Y-axis rotation axes thereof, and to rotate the measurement portions 24 while adjusting the positions where the measurement portions 24 are supported through the X-axis rotation drive portions 29, in cooperation with the horizontal drive portions 27, thereby rotating the measurement portions 24 about the eyeball rotation Y axes of the corresponding subject eyes E. The X-axis rotation drive portions 29 may be configured to rotatably support the measurement portions 24 about X-axis rotation axes thereof, and to rotate the measurement portions 24 while adjusting the positions where the measurement portions 24 are supported, in cooperation with the vertical drive portions 26, thereby rotating the measurement portions 24 about the eyeball rotation X axes of the corresponding subject eyes E.

Thus, the drive mechanisms 23 move the left measurement portion 24L and the right measurement portion 24R either individually or in conjunction in the X, Y, and Z directions, rotate the left measurement portion 24L vertically and horizontally about the eyeball rotation point O of the left subject eye EL, and rotate the right measurement portion 24R vertically and horizontally about the eyeball rotation point O of the right subject eye ER. Thus, the drive mechanisms 23 can move the left and right measurement portions 24L, 24R to desired positions (postures) relative to the subject eyes E.

The drive mechanisms 23 adjust the positions of the left and right measurement portions 24L, 24R, allowing for divergence (divergence movement) or convergence (convergence movement) of the left and right subject eyes EL, ER. In other words, the drive mechanisms 23 (left-eye and right-eye drive mechanisms 23L, 23R) serve as a convergence adjustment mechanism that adjusts a convergence distance Lc. As shown in FIG. 3, the convergence distance Lc refers to a distance from both subject eyes E to a visual line intersection point Is in a plan view of the left and right subject eyes E (EL, ER). The visual line intersection point Is refers to a position (convergence position) where a left-side visual line direction SL (left-side visual line) of the left subject eye EL and a right-side visual line direction SR (right-side visual line) of the right subject eye ER intersect. The left-side visual line direction SL and the right-side visual line direction SR define a convergence angle θc therebetween. Controlling the convergence angle θc sets the convergence distance Lc.

In the ophthalmologic apparatus 1 according to the first embodiment, each of the left and right measurement portions 24L, 24R includes a deflection member 24a (see FIGS. 1 and 2). The left and right measurement portions 24L, 24R obtain, respectively, the eye characteristics of the corresponding subject eyes via the deflection members 24a. The ophthalmologic apparatus 1 adjusts the positions of the left and right measurement portions 24L, 24R so that the deflection members 24a are respectively positioned to correspond to the left and right subject eyes E (EL, ER), thereby allowing for the obtainment of the eye characteristics of both eyes at the same time in a condition in which the examinee keeps both of his/her eyes open (also referred to as binocular vision condition). The ophthalmologic apparatus 1 may adjust the rotational postures of the left and right measurement portions 24L, 24R about the eyeball rotation X axes with the X-axis rotation drive portions 29, thereby allowing for the obtainment of the eye characteristics in a condition in which the subject eyes E are in a downward or upward gaze. The ophthalmologic apparatus 1 may adjust the rotational postures of the left and right measurement portions 24L, 24R about the eyeball rotation Y axes with the Y-axis rotation drive portions 28, thereby allowing the obtainment of the eye characteristics in a condition in which the subject eyes E are in a leftward or rightward gaze.

The examiner's control device 30 is an information processing device that is configured to receive operation commands from the examiner and to output control signals to the controller 40. The examiner's control device 30 may be a portable device such as a tablet terminal or a smartphone and is separatable from the measurement unit 20, allowing the examiner to carry it. The examiner's control device 30 may also be a personal computer such as a laptop personal computer or a desktop personal computer. The examiner's control device 30 may be a controller dedicated to the ophthalmologic apparatus 1. The examiner's control device 30 is configured to exchange information with the controller 40 via wireless communication or network communication.

As shown in FIG. 1, the examiner's control device 30 includes a display 31, an operation controller (not shown), and input buttons (not shown). The display 31 is a touch panel display provided in the examiner's control device 30, which includes input buttons. The operation controller is a built-in microcomputer within the examiner's control device 30. The operation controller is configured to control images displayed on the display 31 based on measurement results and/or detection results from the controller 40. The operation controller outputs the control signals to the controller 40 according to the operation on the input buttons.

The controller 40 is an information processing device located beneath the optometric table 12. The controller 40 is configured to comprehensively control the measurement unit 20 including the left and right measurement optical systems 25L, 25R based on control signals from the examiner's control device 30. The controller 40 transmits the measurement results of the eye characteristics of the left and right subject eyes E (EL, ER) obtained with the left and right measurement portions 24L, 24R to the examiner's control device 30.

The controller 40 according to the present invention can carry out an eyestrain estimation process that estimates the eyestrain of the subject eyes E. In the eyestrain estimation process, the controller 40 firstly controls visual target presenting mechanisms (visual target projection systems 44 described later) to present a left-eye visual target OtL and a right-eye visual target OtR as visual targets Ot, which are described below in detail. The visual targets OtL, OtR are presented to the left and right subject eyes EL, ER at any visual target presentation distance Lp in an identical manner. At this time, the controller 40 controls the drive mechanisms 23 (left-eye and right-eye drive mechanisms 23L, 23R) to adjust the positions and/or orientations of the left and right measurement portions 24L, 24R, thereby setting the convergence angle θc to a predetermined value and adjusting the convergence distance Lc accordingly. This convergence distance Lc may be different from or equal to the visual target presentation distance Lp.

As shown in FIG. 3, the visual target presentation distance Lp refers to the distance along the Z direction from the left subject eye EL to the visual target Ot presented to the left subject eye EL, and the distance along the Z direction from the right subject eye ER to the visual target Ot presented to the right subject eye ER. The visual target presentation distances Lp of the left subject eye EL and the right subject eye ER are set to be the same. In the first embodiment, the visual target projection system 44 (see FIG. 4), which functions as the visual target presenting mechanism, sets the visual target presentation distance Lp. The visual target presentation distance Lp can be calculated from the power of the visual target projection system 44, which refers to the ability of the lens to deflect light beams, and can be expressed in diopter equivalents.

In other words, the controller 40 moves a focusing lens 44e of the visual target projection system 44 to present the visual target Ot at any predetermined distance (visual target presentation distance Lp), based on the far-point position, in accordance with the refractive values (spherical equivalents) of the left and right subject eyes E (EL, ER), thereby controlling the power of the visual target projection system 44. For example, when the visual target presentation distance Lp is set to 50 cm, the controller 40 moves the focusing lens 44e to achieve near vision by adjusting the power difference (2.0 D) when presenting the visual target Ot at the positions corresponding to the far point (0 D) and at 50 cm (2.0 D), relative to the positions corresponding to the far points of the left and right subject eyes EL, ER (e.g., refractive value of left and right subject eyes EL, ER is - 5.0 D). Moving the focusing lens 44e enables changing the power of the visual target projection system 44 and presenting the visual target Ot at a position with a refractive value of -2.0 D. As a result, the controller 40 can present the visual target Ot to both subject eyes E at a perceived position of 50 cm using the visual target projection system 44.

When the controller 40 carries out the eyestrain estimation process, the controller 40 can also set the convergence distance Lc, determined by the convergence angle θc, to be shorter than the visual target presentation distance Lp. At this time, while the maintaining the visual target presentation distance Lp, the controller 40 sets the visual line intersection point Is, determined by the positions and/or orientations of the left and right measurement portions 24L, 24R, to be the near side of (or to be shorter than) the visual target presentation distance Lp. In other words, during the eyestrain estimation process, the controller 40 sets the convergence angle θc1, determined by the positions and/or orientations of the left and right measurement portions 24L, 24R, to be greater than the convergence angle θc2 when binocularly viewing the position where the visual target Ot is presented. This enables the left and right subject eyes EL, ER to perform a convergence movement, such that they focus on an object presented at a distance closer than the visual target presentation distance Lp, and are in an inwardly rotated state (see FIG. 3).

Next, the examinee is instructed to focus on corresponding portions of the left-eye and right-eye visual targets OtL, OtR to achieve binocular fusion. In this state, the controller 40 gradually increases the difference between the brightness (contrast relative to background) of a portion of the left-eye visual target OtL and the brightness (contrast relative to background) of at least a corresponding portion of the right-eye visual target OtR. Once the brightness difference is increased, the controller 40 detects the visual line direction SL of the left subject eye EL and the visual line direction SR of the right subject eye ER, based on the ocular information (anterior ocular segment image E' (see FIG. 4)) obtained by using an ocular information obtaining portion (anterior ocular segment observation system 45 described later) and the rotational angles of the left and right measurement portions 24L, 24R.

In order to detect the visual line directions SL, SR, the controller 40 first determines the two-dimensional positions of the pupil centers of the left and right subject eyes EL, ER, based on the anterior ocular segment images E' and magnification of the left and right subject eyes EL, ER and the rotational angles of the left and right measurement portions 24L, 24R controlled by the drive mechanisms 23. Next, the controller 40 determines the reference positions, which are the two-dimensional positions of the bright spots (bright spot images Br) visualized with the XY alignment systems 42, based on the anterior ocular segment images E' of the left and right subject eyes EL, ER and their magnification, as well as the rotational angles of the left and right measurement portions 24L, 24R controlled by the drive mechanisms 23. Then, the controller 40 determines the visual line directions SL, SR, based on the reference positions and the pupil center positions. The method for determining the visual line directions SL, SR is not limited to the above method, and another known method may be used. The controller 40 estimates the strain level (eyestrain) of the left and right subject eyes EL, ER, based on the visual line directions SL, SR of the left and right subject eyes EL, ER.

When the controller 40 detects the visual line directions SL, SR of the left and right subject eyes EL, ER during the eyestrain estimation process, the controller 40 detects the focus points (accommodation positions) of the left and right subject eyes EL, ER, respectively, based on the ocular information (a ring image formed by light reflected from the ocular fundus) obtained by using the ocular information obtaining portion (refractive power measurement projection system 46 and refractive power measurement light receiving system 47 described later). Each focus point is represented by the refractive power of the left or right subject eye EL, ER when observing an object at a predetermined position.

The detailed configurations of the left and right measurement optical systems 25L, 25R will be described with reference to FIG. 4. The left and right measurement optical systems 25L, 25R have the same configuration. Accordingly, only the left measurement optical system 25L is described, and the description of the right measurement optical system 25R is omitted in this specification.

The left measurement optical system 25L is an optical system that presents any visual target (including the visual target Ot described later) to the left subject eye EL and examines it. The left measurement optical system 25L includes a Z alignment system 41, an XY alignment system 42, a keratometry measurement system 43, a visual target projection system 44, an anterior ocular segment observation system 45, a refraction measurement projection system 46, and a refraction measurement light receiving system 47. In the following description, an ocular fundus conjugate position P and a pupil conjugate position Q are used. The ocular fundus conjugate position P means a position optically substantially conjugate to an ocular fundus Ef of the left subject eye EL, which can include an exact position optically conjugate to the ocular fundus Ef and any proximal positions near the exact position, in an aligned state. The pupil conjugate position Q means a position optically substantially conjugate to a pupil of the left subject eye EL, which can include an exact position optically conjugate thereto and any proximal positions near the exact position, in an aligned state.

The Z alignment system 41 is configured to project light (infrared light in the first embodiment) onto the left subject eye EL for the alignment purpose in the direction of the optical path (front or back direction) of the anterior ocular segment observation system 45. The Z alignment system 41 includes a Z alignment light source 41a configured to emit light. The light from the Z alignment light source 41a is turned into a parallel light flux by using the projecting lens 41b and then is projected onto the cornea Ec of the left subject eye EL through an alignment hole formed through a keratometric plate 43a. Based on a bright spot projected onto the cornea Ec, the controller 40 or the examiner moves the left measurement optical system 25L in the Z direction by moving the left measurement portion 24L (right measurement portion 24R) such that the ratio of the distance between two spot images by the Z alignment light source 41a on the imaging element 45h of the anterior ocular segment observation system 45 and the diameter of the keratometric ring image should fall in a predetermined range. This places the left measurement optical system 25L at a proper position in the optical axis direction relative to the left subject eye EL.

The XY alignment system 42 is configured to illuminate light (infrared light in the first embodiment) onto the left subject eye EL for the alignment purpose in the X and Y directions orthogonal to the optical axis (Z-axis) of the anterior ocular segment observation system 45. The XY alignment system 42 includes an XY alignment light source 42a and a projecting lens 42b in an optical path branched from the anterior ocular segment observation system 45 by using the half mirror 45c. The XY alignment system 42 makes the light emitted from the XY alignment light source 42a proceed to the anterior ocular segment observation system 45 through the projecting lens 42b. Then, the XY alignment system 42 reflects the light with the half mirror 45c and then projects the reflected light onto the left subject eye EL through the anterior ocular segment observation system 45. The reflected light from the cornea Ec of the left subject eye EL is guided to the imaging element 45h through the anterior ocular segment observation system 45. Based on a bright spot projected onto the cornea Ec, the controller 40 or the examiner moves the left measurement portion 24L (right measurement portion 24R) in the vertical direction or the horizontal direction, thereby conducting an alignment in directions (Y direction (vertical) and X direction (horizontal)) orthogonal to the optical axis L of the anterior ocular segment observation system 45. Thereby, the left measurement optical system 25L is placed at a proper position in the X and Y directions relative to the left subject eye EL. The alignment method in each of X, Y, and Z directions is not limited to the method using the Z alignment system 41 and the XY alignment system 42. The alignment method may be ones capable of measuring the position of the subject eye E, for example, by using a stereo camera in the ophthalmologic apparatus 1.

The image (bright spot image Br) based on the reflected light from the cornea Ec is formed in a manner to overlap the anterior ocular segment image E'. The controller 40 is configured to control the display of the anterior ocular segment image E' including the bright spot image Br and the alignment mark on the display 31. With the manual XY alignment, the examiner operates the examiner's control device 30 to move the left measurement optical system 25L in the X and Y directions and to guide the bright spot image within the alignment mark. With the automatic XY alignment, the controller 40 moves the left measurement portion 24L (right measurement portion 24R) to cancel the displacement of the bright point image relative to the alignment mark, thereby moving the left measurement optical system 25L in the X and Y directions.

The keratometry measurement system 43 is configured to project a ring-shaped light flux (infrared light) onto the cornea Ec for measuring the shape of the cornea Ec of the left subject eye EL. The keratometry measurement system 43 includes the keratometric plate 43a disposed between the objective lens 45a and the left subject eye EL, and a keratometric ring light source 43b provided on the back side (side of the objective lens 45a) of the keratometric plate 43a. The keratometry measurement system 43 projects the ring-shaped light flux on the cornea Ec of the left subject eye EL as light from the keratometric ring light source 43b passes through a slit of the keratometric plate 43a. The imaging element 45h detects the reflected light (keratometric ring image) from the cornea Ec of the left subject eye EL together with the anterior ocular segment image E'. The controller 40 calculates the cornea Ec shape parameter representing the shape of the cornea Ec with a known calculation based on the keratometric ring image.

The visual target projection system 44 sets the visual target presentation distance Lp to any distance and presents various visual targets, such as fixation targets, visual targets for the subjective examination, and the visual target Ot, to the left subject eye EL. The visual target projection system 44 includes a display 44a, a half mirror 44b, a relay lens 44c, a reflective mirror 44d, a focusing lens 44e, a relay lens 44f, a field lens 44g, a variable cross-cylinder lens (VCC) 44h, a reflective mirror 44k, and a pinhole plate 44m. The visual target projection system 44 shares a dichroic mirror 46h with the refraction measurement projection system 46. In addition, the visual target projection system 44 shares a dichroic mirror 45b and the objective lens 45a with the anterior ocular segment observation system 45. The visual target projection system 44 further includes at least two glare light sources 44n, 44n, which illuminate the left subject eye EL with glare light, at a position around the optical axis and on an optical path different from the optical path to elements such as the display 44a which displays various visual targets.

The display 44a functions as a visual target presenting portion that presents various visual targets. The display 44a is provided at the ocular fundus conjugate position P on the optical path of the visual target projection system 44. The display 44a is configured to display fixation targets and point-like visual targets for stabilizing the line of sight during the objective examination or when applying fogging to the left subject eye EL. The display 44a is also configured to display the subjective examination visual targets for subjectively examining eye characteristics (e.g., visual acuity value, far-point power, and near-point power) of the left subject eye EL. The display 44a may use an electroluminescence (EL) display, a liquid crystal display (LCD), or other types of displays. The display 44a is configured to display the visual target with a desired shape or form and a desired contrast (brightness). The display 44a is also configured to display various visual targets and to adjust the brightness of the displayed visual targets (contrast of the visual target relative to the background) under the control of the controller 40. In the first embodiment, the controller 40 controls the display 44a to display the left-eye visual target OtL (or the right-eye visual target OtR in the right measurement optical system 25R), which serves as the visual target Ot (see FIG. 5, etc.) used for the eyestrain estimation process, and to adjust the brightness of a portion thereof as necessary.

The visual target projection system 44 reflects the light from the display 44a with the half mirror 44b, passes the light through the relay lens 44c, reflects it with the reflective mirror 44d, and then passes it through the focusing lens 44e. Subsequently, the visual target projection system 44 passes the light through the relay lens 44f, aligns its travel direction using the field lens 44g, passes it through the VCC 44h, reflects it with the reflective mirror 44k, passes it through the dichroic mirror 46h, and then reflects it with the dichroic mirror 45b. Next, the visual target projection system 44 projects the light reflected by the dichroic mirror 45b through the objective lens 45a onto the ocular fundus Ef.

The controller 40 controls a drive motor (not shown) to drive the focusing lens 44e to move forward and backward in the optical axis direction. The controller 40 moves the focusing lens 44e toward the left subject eye EL to adjust the spherical power of the left subject eye EL to the negative diopter side (-D side), that is, to adjust the refractive power thereof to the negative side. Also, the controller 40 moves the focusing lens 44e in a direction away from the left subject eye EL to adjust the spherical power of the left subject eye EL to the positive diopter side (+D side), that is, to adjust the refractive power thereof to the positive side (toward the distant vision direction). Furthermore, the controller 40 controls the forward and backward movement of the focusing lens 44e to adjust the presentation position of the visual target displayed on the display 44a, that is, the value of the presentation distance Lp from the left subject eye EL to the visual target presentation position to any value. The focusing lens 44e is configured to move together with a refraction measurement light source 46a of the refraction measurement projection system 46 and a focusing lens 47d of the refraction measurement light receiving system 47.

For the subjective examination, the controller 40 controls the focusing lens 44e to move in the optical axis direction based on the result of the objective examination, thereby controlling the visual target presentation distance Lp and the spherical power of the left subject eye EL. The controller 40 controls the display 44a to present a predetermined visual target, which is selected by a user of the ophthalmologic apparatus 1, including the examiner to present the predetermined visual target to the examinee at a predetermined visual target presentation distance Lp relative to the left subject eye EL adjusted to having a predetermined spherical power. When the examinee responds to the subjective examination of the visual target, the controller 40 receives the input of the response. For example, in the visual acuity measurement, the controller 40 selects the next visual target based on the examinee's response to the visual target such as the Landolt ring, displays the selected next visual target to the examinee, and determines the visual acuity value by repeating this process. Accordingly, the visual target projection system 44 functions as a subjective examination system.

Based on these facts, the ophthalmologic apparatus 1 can separately present the visual targets (including the left-eye visual target OtL) to the left subject eye EL and can set the visual target presentation distance Lp to a desired distance. Accordingly, the display 44a of the left measurement optical system 25L serves as a display for the left eye, presenting the left-eye visual target OtL. The same applies to the right measurement optical system 25R, where the display 44a thereof serves as a display for the right eye, presenting the right-eye visual target OtR. Thus, the ophthalmologic apparatus 1 is configured to individually and partially or entirely adjust the brightness of the visual target presented to the left subject eye EL and that of the visual target presented to the right subject eye ER, and to increase the brightness difference between them as necessary.

The visual target projection system 44 includes the pinhole plate 44m at the pupil conjugate position Q. In the first embodiment, the pinhole plate 44m is provided between the field lens 44g and the VCC 44h. This pinhole plate 44m is formed by making a through hole in a plate member. The controller 40 controls the pinhole plate 44m to be inserted into and removed from the optical path of the visual target projection system 44. When the pinhole plate 44m is inserted into the optical path, the through hole is positioned on the optical axis. Inserting the pinhole plate 44m into the optical path during the subjective examination allows for a pinhole test that determines if the left subject eye EL can be corrected with glasses. Furthermore, the pinhole plate 44m is also inserted into the optical path to measure the convergence adjustment, which is described later. The pinhole plate 44m is not limited to the configuration in the first embodiment. The pinhole plate 44m may be provided at a position that is substantially conjugate to the pupil of the left subject eye EL in the optical path.

The anterior ocular segment observation system 45 is configured to observe the anterior ocular segment of the left subject eye EL and capture images thereof. The anterior ocular segment observation system 45 includes an anterior ocular segment illumination light source 48 that is configured to illuminate an illumination light (infrared light in the first embodiment) onto the anterior ocular segment of the left subject eye EL. The anterior ocular segment observation system 45 allows the light reflected from the anterior ocular segment of the left subject eye EL to pass through the objective lens 45a, to be transmitted through the dichroic mirror 45b and the half mirror 45c, to pass through the relay lens 45d and the relay lens 45e, and to be transmitted through the dichroic mirror 45f. The anterior ocular segment observation system 45 allows the light to form an image on the imaging surface of the imaging element 45h by using the imaging lens 45g. The imaging surface of the imaging element 45h is at the pupil conjugate position Q. As a result, on the imaging element 45h, a keratometry ring image, a light flux of the Z alignment light source 41a, and a light flux (bright spot image Br) of the XY alignment light source 42a are projected to form the anterior ocular segment image E'. The imaging element 45h captures images to output a signal at a predetermined rate and also outputs the image signal to the controller 40. The controller 40 controls the display 31 of the examiner's control device 30 to display the anterior ocular segment image E' (moving image), which is based on the image signal output from the imaging element 45h, on the display 31. The controller 40 can also detect the visual line direction SL of the left subject eye EL based on the anterior ocular segment image E' and the rotational angle of the left measurement portion 24L.

The refraction measurement projection system 46 and the refraction measurement light receiving system 47 constitute an objective measurement optical system used for the objective refraction measurement, which objectively measures the refraction values (refractive characteristics) as the eye characteristics of the left subject eye EL. The refraction measurement projection system 46 projects a ring-shaped light flux (infrared light) for objective measurement from the refraction measurement light source 46a onto the ocular fundus Ef. The refraction measurement light receiving system 47 receives the returning light of the ring-shaped light flux from the left subject eye EL. The configuration of the refraction measurement projection system 46 and the refraction measurement light receiving system 47 is not limited to that of the first embodiment, as long as it achieves the projection of the measurement light flux onto the ocular fundus Ef of the left subject eye EL and the acquisition of the measurement light flux reflected by the ocular fundus Ef as a measurement ring image. One example of an alternative configuration of the refraction measurement projection system 46 and the refraction measurement light receiving system 47 includes projecting a point-shaped spotlight as the measurement light flux onto the ocular fundus Ef and converting the measurement light flux reflected by the ocular fundus Ef (reflected light flux) into a ring-shaped light flux by passing it through a ring-shaped slit and/or a lens to obtain the measurement ring image.

In the first embodiment, the refraction measurement light source 46a is implemented with a super luminescent diode (SLD) light source, which is a high-intensity light source with an emission diameter less than a predetermined size. The refraction measurement light source 46a is configured to move in the optical axis direction in conjunction with the focusing lens 44e and the focusing lens 47d and is disposed at the ocular fundus conjugate position P. A ring diaphragm 46e is a transmissive portion formed in a ring-shaped and is disposed at the pupil conjugate position Q. The focusing lens 47d is configured to move in the optical axis direction in conjunction with the refraction measurement light source 46a and the focusing lens 44e. The focusing lens 47d may be a known variable focusing lens, which can adjust its focus point under the control of the controller 40. The optical system of the refraction measurement light receiving system 47 disposes the imaging surface of the imaging element 45h at the ocular fundus conjugate position P.

The refraction measurement projection system 46 allows the light emitted from the refraction measurement light source 46a to pass through a relay lens 46b and to enter onto a cone surface of a conical prism 46c. The refraction measurement projection system 46 allows the light entered onto the cone surface to be deflected, to be emitted from the bottom surface of the conical prism 46c, and to pass through a field lens 46d and a ring diaphragm 46e (its transmissive portion). The refraction measurement projection system 46 allows the light (ring-shaped light flux) to be reflected off a reflection surface of a holed prism 46f, to pass through a rotary prism 46g, and to be reflected off a dichroic mirror 46h. The refraction measurement projection system 46 allows the reflected light to be reflected off the dichroic mirror 45b, to pass through the objective lens 45a, and to be projected onto the left subject eye EL.

It is preferable to dispose the conical prism 46c at a position as close as possible to the pupil conjugate position Q. The conical prism 46c may include a ring diaphragm 46e attached to its bottom surface facing the field lens 46d. **In** this case, for example, the bottom surface of the conical prism 46c may be deposited with a light shielding film to form a ring-shaped light transmitting portion. Alternatively, the ring diaphragm 46e may be on the conical surface side of the conical prism 46c.

The field lens 46d may include the ring diaphragm 46e affixed to the lens surface on the side of the left subject eye EL, for example. **In** this case, a light shielding film may be deposited on the lens surface of the field lens 46d so that a ring-shaped transmissive portion is formed. The refraction measurement projection system 46 may have a configuration in which the field lens 46d is omitted. The ring diaphragm 46e may be formed with the light transmitting portion having a shape corresponding to a predetermined measurement pattern, at a position eccentric to (offset from) the optical axis of the refractometry projection system 6. The number of the light transmitting portions may be one or more than two. The rotary prism 46g is used for averaging the light amount distribution of the ring-shaped luminous flux with respect to the disease site and the blood vessel of the fundus Ef and reducing speckle noise caused by the light source.

The refraction measurement light receiving system 47 allows the return light of the ring-shaped light flux projected onto the ocular fundus Ef to pass through the objective lens 45a and to be reflected off the dichroic mirror 45b and the dichroic mirror 46h. The refraction measurement light receiving system 47 allows the reflected return light to pass through the rotary prism 46g, the hole of the holed prism 46f, and the relay lens 47a, to be reflected off the reflective mirror 47b, and to pass through the relay lens 47c and the focusing lens 47d. The refraction measurement light receiving system 47 allows the light, which has passed therethrough, to be reflected off a reflective mirror 47e, to pass through the dichroic mirror 45f, and to form an image on the imaging surface of the imaging element 45h with the imaging lens 45g.

The controller 40 calculates the parameter of the ocular refractive power by using any known calculation, based on the output from the imaging element 45h. The parameter of the ocular refractive power includes refractive value (refractive power), spherical power, astigmatism power, and astigmatic axis angle of the left and right subject eyes EL, ER. Based on the control signals from the examiner's control device 30, the controller 40 comprehensively controls the left and right measurement optical systems 25L, 25R (each of which includes systems such as the refraction measurement projection system 46, the refraction measurement light receiving system 47, the visual target projection system 44), and the measurement unit 20. The controller 40 sends the measurement results of the eye characteristics of the left and right subject eyes EL, ER measured by using the measurement head 22 to the examiner's control device 30.

Next, the visual target Ot used in the eyestrain estimation process will be described with reference to FIGS. 5-7. The visual target Ot includes the left-eye visual target OtL and the right-eye visual target OtR. The left-eye visual target OtL is presented to the left subject eye EL by using the left measurement optical system 25L, and the right-eye visual target OtR is presented to the right subject eye ER by using the right measurement optical system 25R. In the first embodiment, the visual target Ot is formed by displaying a white sign on a black background on each display 44a of the left and right measurement optical systems 25L, 25R. As shown in FIG. 5, the left-eye visual target OtL and the right-eye visual target OtR each include a first visual target sign s1 and a second visual target sign s2, which are identical in shape and size. Hereinafter, when referring to them individually, the first visual target sign s1 and the second visual target sign s2 of the left-eye visual target OtL are denoted as the left-side first visual target sign s1L and the left-side second visual target sign s2L, while those of the right-eye visual target OtR are denoted as the right-side first visual target sign s1R and the right-side second visual target sign s2R.

Each of the first visual target signs s1 is an asterisk mark presented at the center of a rectangular display 44a such as a liquid crystal panel. Each of the second visual target signs s2 is an annular mark that surrounds the first visual target sign s1. When both second visual target signs s2 are fused binocularly, the convergence angle θc becomes smaller, and the convergence distance Lc increases (appears farther) compared to when both first visual target signs s1 are fused binocularly. More specifically, the left-side second visual target sign s2L has a center position that is displaced outward (toward the left in FIG. 5) from the left-side first visual target s1L, while the right-side second visual target sign s2R has a center position that is displaced outward (toward the right in FIG. 5) from the right-side first visual target sign s1R. The amount of the outward displacement of the left-side second visual target sign s2L from the center position of the display 44a is equal to that of the right-side second visual target sign s2R from the center position of the display 44a. Accordingly, the left-eye and right-eye visual targets OtL, OtR are arranged such that the first visual target signs s1 and the second visual target signs s2, which are identical in design, size, and line width, are left-right reversed versions of each other. The design, size, and line width of each of the first and second visual target signs s1, s2 are determined to provide an appropriate binocular fusion stimulus to the left and right subject eyes EL, ER during the binocular fusion where the images projected onto the retina of the left and right subject eyes EL, ER are integrated into a single image when viewed binocularly.

The left-eye and right-eye visual targets OtL, OtR are designed such that, when the binocular fusion is achieved by focusing on the first visual target signs s1 in the binocular vision, the second visual target signs s2 appear as two overlapping images on either side of the single first visual target sign s1 as shown in FIG. 6. Furthermore, the left-eye and right-eye visual targets OtL, OtR are designed such that, when the binocular fusion is achieved by focusing on the second visual target signs s2 in the binocular vision, the second visual target sign s2 appears as a single image with two first visual target signs s1 positioned side by side inside it as shown in FIG. 7.

Next, the structure and procedural flow of the eyestrain estimation process by the ophthalmologic apparatus 1 will be described with reference to the flowchart illustrated in FIG. 8. This process begins when the mode for estimating the eyestrain of the subject eyes is selected in the ophthalmologic apparatus 1.

In Step S1, the controller 40 measures the ocular refractive power, and then the process proceeds to Step S2. Specifically, in Step S1, the controller 40 measures the ocular refractive power of the left and right subject eyes EL, ER. More specifically, the controller 40 first aligns the left measurement portion 24L relative to the left subject eye EL and the right measurement portion 24R relative to the right subject eye ER. Next, the controller 40 executes fogging control while the left and right subject eyes EL, ER fixate on the fixation targets. Then, the controller 40 measures the ocular refractive power of each of the left and right subject eyes EL, ER based on the ring image formed from the ocular fundus reflection light, which is obtained using the refraction measurement projection system 46 and the refraction measurement light receiving system 47. The position of the focusing lens 44e in the visual target projection system 44 is adjusted based on the measured ocular refractive power so that the spherical power of the left and right subject eyes EL, ER is in a fully corrected state when the examination distance is set at the far-point position. Subsequently, the controller 40 may measure the far-point position in detail through a subjective eye examination and determine the position of the focusing lens 44e in the visual target projection system 44.

In Step S2, the visual target Ot is presented, and the process proceeds to Step S3. In Step S2, the controller 40 controls the visual target projection system 44 of the left measurement optical system 25L to present the left-eye visual target OtL on the display 44a, and the visual target projection system 44 of the right measurement optical system 25R to present the right-eye visual target OtR on the display 44a. At this time, the controller 40 adjusts the left-eye and right-eye visual targets OtL, OtR so that they are presented at an equal distance as the visual target presentation distance Lp from the left and right subject eyes EL, ER and have the same brightness (contrast of the visual target relative to the background). The controller 40 controls the drive mechanism 23 (left-eye and right-eye drive mechanisms 23L, 23R) to adjust the positions (and/or orientations) of the left and right measurement portions 24L, 24R and to set the convergence angle θc of the visual target Ot to any angle. In the first embodiment, the visual target presentation distance Lp is set to 33 cm, and the convergence angle θc is adjusted from a state where the convergence distance Lc is equal to the visual target presentation distance Lp, further inward by 20 Δ (prism) (10 Δ for each of the left and right subject eyes EL, ER). At this time, the convergence distance Lc at the binocular fusion of the second visual target signs s2 is controlled so that it does not exceed the visual target presentation distance Lp. The visual target presentation distance Lp and the convergence angle θc are not limited to the configuration of the first embodiment but may be suitably adjusted according to the examinee's age, eyesight, and other factors. Additionally, in the ophthalmologic apparatus 1 according to the first embodiment, when the controller 40 presents the visual targets Ot, it also displays the anterior ocular segment images E' of the left and right subject eyes EL, ER, obtained by the anterior ocular segment observation systems 45, on the display 31 of the examiner's control device 30. The presentation of the anterior ocular segment images E' continues until the eyestrain estimation process is completed.

In Step S3, the visual line directions SL, SR, which serve as reference directions, are detected, and then the process proceeds to Step S4. In Step S3, the examinee performs the binocular fusion of the corresponding portions of the visual targets Ot presented in Step S2, and the visual line directions SL, SR are detected under that condition. In the first embodiment, once the examinee focuses on the first visual target signs s1 and the binocular fusion is confirmed, the controller 40 detects the left-side visual line direction SL as the reference direction of the left subject eye EL, and the right-side visual line direction SR as the reference direction of the right subject eye ER. Thus, the visual line directions SL, SR, which serve as the reference directions, correspond to the visual line directions of the left and right subject eyes EL, ER when the examinee performs the binocular fusion of the first visual target signs s1, which are corresponding portions of the visual targets Ot in this case. These visual line directions SL, SR are detected based on the anterior ocular segment images E' of the left and right subject eyes EL, ER, which are obtained by the anterior ocular segment observation system 45, and the rotational angles of the left and right measurement portions 24L, 24R, which are controlled by the drive mechanisms 23. Accordingly, the anterior ocular segment observation system 45 functions as an ocular information obtaining portion that obtains ocular information for detecting the visual line directions SL, SR of the left and right subject eyes EL, ER. In the ophthalmologic apparatus 1 according to the first embodiment, the controller 40 displays the visual line directions SL, SR (and their information), which serve as the reference directions, as the detection results on the display 31 of the examiner's control device 30.

Here, in Step S3, when the binocular fusion cannot be confirmed, the process of obtaining the visual line directions SL, SR as reference directions is repeated by instructing the examinee refocus on the first visual target signs s1. The lack of the binocular fusion can be determined by a large difference between the convergence angle θc derived from the obtained visual line directions SL, SR and the convergence angle θc set in Step S2, and/or by the examinee reporting that the first visual target signs s1 appear double. If repeating the detection of the visual line directions SL, SR does not improve the situation, the controller 40 may return to Step S2 to adjust the visual target presentation distance Lp and/or the convergence angle θc, and then the process proceeds again to Step S3. At this time, the binocular fusion can be facilitated by the following approaches, such as increasing the visual target presentation distance Lp, decreasing the convergence angle θc, bringing closer the visual target presentation distance Lp and the convergence distance Lc determined by the convergence angle θc, and setting the visual target presentation distance Lp and the convergence distance Lc based on the heterophoria angle of the subject eye E.

In Step S4, the brightness difference is increased, and then the process proceeds to Step S5. In Step S4, the controller 40 controls the left and right displays 44a to increase the brightness difference (contrast difference) between the brightness of the left-side first visual target sign s1L of the left-eye visual target OtL and that of the right-side first visual target sign s1R of the right-eye visual target OtR by a predetermined amount. In Step S4 of the first embodiment, the brightness difference is increased by lowering the brightness of one of the left-side and right-side first visual target signs s1L, s1R while maintaining the brightness of the other. This brightness difference may be increased continuously or stepwise over time. Accordingly, the brightness difference (contrast difference) between the left-side and right-side first visual target signs s1L, s1R increases continuously or stepwise over time, making it progressively more difficult to see one of the left-side and right-side first visual target signs s1L, s1R whose brightness is lowered.

In Step S5, the visual line directions SL, SR are detected, and then the process proceeds to Step S6. In Step S5, similar to Step S3, the controller 40 detects the visual line directions SL, SR of the left and right subject eyes EL, ER. The controller 40 displays the visual line directions SL, SR (and their information) as the detection results on the display 31 of the examiner's control device 30.

In Step S6, the controller 40 detects the focus point, and then the process proceeds to Step S7. Specifically, in Step S6, the controller 40 detects the focus point (ocular refractive value when observing an object at a predetermined position) of the left subject eye EL and that of the right subject eye ER. The controller 40 detects each of the focus points, based on the ring image formed from the ocular fundus reflection light, which is obtained using the refraction measurement projection system 46 and the refraction measurement light receiving system 47. In other words, the controller 40 determines the focus point (accommodation position) on the visual axis of the left or right subject eye EL, ER from the refractive power thereof. Accordingly, the refraction measurement projection system 46 and the refraction measurement light receiving system 47 function as the ocular information obtaining portion configured to obtain the ocular information for detecting the focus points of the left and right subject eyes EL, ER.

In Step S7, the controller 40 determines whether the binocular fusion is disrupted. If the binocular fusion is disrupted (YES), the process proceeds to Step S8. If the binocular fusion is not disrupted (NO), the process returns to Step S4. In Step S7, the controller 40 determines whether the binocular fusion is disrupted based on the visual line directions SL, SR, which are the reference directions, detected in Step S3, the visual line directions SL, SR detected in Step S5, and the focus points of the left and right subject eyes E (EL, ER) detected in Step S6. In the first embodiment, the controller 40 determines the change in the visual line directions SL, SR (ocular position displacement) based on whether or not the visual line directions SL, SR are displaced by at least a predetermined amount (e.g., ± 0.5°) relative to the reference visual line directions SL, SR. If such a change occurs, the controller 40 determines that the binocular fusion of the first visual target signs s1 is disrupted. Alternatively, the controller 40 may also determine that the binocular fusion is disrupted if the controller 40 detects that the examinee has shifted the visual line directions SL, SR toward the second visual target signs s2. Whether the visual line directions SL, SR have shifted toward the second visual target signs s2 may be determined based on the positions of the second visual target signs s2 on the displays 44a, as well as the visual target presentation distance Lp and the convergence angle θc of the presented visual targets Ot. Furthermore, in the first embodiment, the controller 40 determines that the binocular fusion of the first visual target signs s1 is disrupted when the focus points of the left and right subject eyes EL, ER deviate from the visual target presentation distance Lp of the visual targets Ot by at least a predetermined amount (e.g., ± 1.0 D as a diopter conversion value).

As described above, the controller 40 repeatedly executes the procedures from Steps S4 to S7 until it determines that the binocular fusion is disrupted in Step S7. Accordingly, the brightness difference between the left-side first visual target sign s1L of the left-eye visual target OtL and the right-side first visual target sign s1R of the right-eye visual target OtR gradually increases over time until the binocular fusion of the first visual target signs s1 is disrupted. At this time, the display 31 continues to display the anterior ocular segment images E' of the left and right subject eyes EL, ER and the detection results of the visual line directions SL, SR. This allows the examiner to monitor the condition of the left and right subject eyes E (EL, ER) in real time.

**In** Step S8, the brightness difference at the moment when the binocular fusion is disrupted (also referred to as the brightness difference at the moment of the binocular fusion disruption) is determined, and then the process proceeds to Step S9. Specifically, in Step S8, the controller 40 obtains the brightness difference between the left-side first visual target sign s1L and the right-side first visual target sign s1R at the moment when the binocular fusion is determined to be disrupted in Step S7. The controller 40 appropriately displays the obtained brightness difference at the moment of the binocular fusion disruption on the display 31 of the examiner's control device 30.

**In** Step S9, the eyestrain is estimated, and the eyestrain estimation process is then completed. In Step S9, the controller 40 estimates the eyestrain of the left and right subject eyes E (EL, ER), based on information about the brightness difference at the moment of the binocular fusion disruption, which was determined in Step S8, and then appropriately displays the estimation results on the display 31 of the examiner's control device 30. For example, if the brightness difference at the moment of the binocular fusion disruption in the current measurement is smaller (i.e., if the binocular fusion is disrupted earlier) than in the previous measurements at different times on the same examinee, the controller 40 determines that the eyestrain level in the current measurement is higher than in the previous measurement. Alternatively, the controller 40 may determine the eyestrain level by comparing the brightness difference at the moment of the binocular fusion disruption in the current measurement with general brightness differences previously collected from other examinees. Furthermore, the controller 40 may determine the eyestrain level in the current measurement based on the relationship between the previously collected brightness difference at the moment of the binocular fusion disruption from multiple examinees and their corresponding eyestrain levels.

Next, the flow of the above eyestrain estimation process will be described. The eyestrain estimation process starts when the examinee sets his/her face at a predetermined position to face the ophthalmologic apparatus 1 and when the examiner selects the mode for estimating the eyestrain level of the subject eyes. The process then proceeds in the sequence of Steps S1, S2, and S3. Thus, after measuring the refractive power of the left and right subject eyes E (EL, ER), the controller 40 presents the visual targets Ot at a predetermined visual target presentation distance Lp and a predetermined convergence angle θc. Then, the controller 40 detects the visual line directions SL, SR, which are the reference directions, for the left and right subject eyes E (EL, ER) under the condition of the binocular fusion of the first visual target signs s1 used as the corresponding portions of the visual targets Ot.

Then, the eyestrain estimation process proceeds in the sequence of Steps S4, S5, and S6. The controller 40 increases the brightness difference between the left-side first visual target sign s1L and the right-side first visual target sign s1R and detects the visual line directions SL, SR, and the focus points of the left and right subject eyes E (EL, ER). Next, the eyestrain estimation process proceeds to Step S7, and the controller 40 determines whether the binocular fusion is disrupted based on the visual line directions SL, SR as the reference directions, the current visual line directions SL, SR, and the focus points. The eyestrain estimation process repeats the sequence of Steps S4, S5, S6, and S7 until the controller 40 determines that the binocular fusion is disrupted. Once the controller 40 determines that the binocular fusion is disrupted, the process proceeds to the sequence of Steps S8 and S9. At this point, the controller 40 determines the brightness difference at the moment of the binocular fusion disruption and estimates eyestrain based on the determined brightness difference. It should be noted that the brightness reduction may be applied to only one of the subject eyes E (e.g., the non-dominant eye) or sequentially to both subject eyes.

In this way, the eyestrain estimation process increases the brightness difference between the left-side first visual target sign s1L and the right-side first visual target sign s1R while maintaining the binocular fusion of these signs and then estimates eyestrain based on the brightness difference at the moment of the binocular fusion disruption. Accordingly, the eyestrain estimation process is required to obtain the brightness difference at the moment of the binocular fusion disruption by accurately detecting the timing of the binocular fusion disruption. The eyestrain estimation process detects the current visual line directions SL, SR whenever the controller 40 increases the brightness difference and then checks whether there is a change (ocular position displacement) in the current visual line directions SL, SR from the reference visual line directions SL, SR to detect the timing of the binocular fusion disruption. The eyestrain estimation process detects the current visual line directions SL, SR based on the anterior ocular segment images E', which show the actual condition of the subject eyes in real time, obtained by the anterior ocular segment observation system 45, and on the rotational angles of the left and right measurement portions 24L, 24R. This enables the process to accurately detect the timing of the binocular fusion disruption, compared with detection based on the response from the examinee.

As described above, the eyestrain estimation process utilizes the change (ocular position displacement) in the visual line directions SL, SR of the left and right subject eyes E (EL, ER) from the left-side and right-side first visual target signs s1L, s1R to other directions when the binocular fusion of the first visual target signs s1 is disrupted. In other words, if the binocular fusion of the first visual target signs s1 is disrupted but the visual line directions SL, SR do not deviate from the left-side and right-side first visual target signs s1L, s1R, the disruption of the binocular fusion cannot be detected.

When the visual targets Ot are configured with only the first visual target signs s1, the following issue may arise. Here, for simplification of the description, it is assumed that the brightness of the right-side first visual target sign s1R is reduced to increase the brightness difference between the left-side first visual target sign s1L and the right-side first visual target sign s1R. As the brightness of the right-side first visual target sign s1R is reduced, it becomes increasingly difficult for the right subject eye ER to see and eventually to recognize the right-side first visual target sign s1R. As the right subject eye ER loses its visual target, its viewing condition of the first visual target signs s1 is disrupted, causing the right-side visual line direction SR to shift away from the first visual target sign s1. Here, when the visual targets Ot are configured with only the first visual target signs s1, the right-side visual line direction SR may remain unchanged from the state in which the right-side visual line direction SR is directed to the first visual target sign s1 because there is no object to be seen due to the difficulty in visually recognizing the right-side first visual target sign s1R. This results in the failure to detect any change in the right-side visual line direction SR, making it impossible to properly determine the timing of the binocular fusion disruption.

The display 44a displays the visual targets Ot. When the visual targets Ot are formed by displaying white signs on a black background as in the first embodiment, the edge of the display 44a becomes noticeable when the brightness of the right-side first visual target sign s1R is lowered to the point of being nearly turned off. This occurs because lowering the brightness of the right-side first visual target sign s1R darkens the entire screen, causing the edge of the display 44a to appear faintly illuminated due to the influence of the backlight. As a result, the edge of the display 44a acts as a binocular fusion stimulus. If the right-side visual line direction SR (center position) toward the right-side first visual target sign s1R and that toward the edge of the display 44a are the same, the binocular fusion disruption of the first visual target signs s1 does not cause a change in the right-side visual line direction SR, making it impossible to detect the timing of the binocular fusion disruption.

In contrast, the ophthalmologic apparatus 1 according to the present invention is configured such that the visual targets Ot each include the first visual target sign s1 and the second visual target sign s2, which has a different parallax from the first visual target sign s1. In the eyestrain estimation process, the ophthalmologic apparatus 1 increases only the brightness difference between the visual target signs that the subject eyes E are directed at, either the first visual target signs s1 or the second visual target signs s2. As a result, once the binocular fusion of the first visual target signs s1 is disrupted, the visual line directions SL, SR of the subject eyes E shift toward the second visual target signs s2. The details are as follows. For simplification of the description, it is assumed that the brightness of the right-side first visual target sign s1R is reduced to increase the brightness difference between the left-side first visual target sign s1L and the right-side first visual target sign s1R.

FIG. 9 shows a condition in which the ophthalmologic apparatus 1 presents the left and right visual targets OtL, OtR to the left and right subject eyes EL, ER, respectively, thereby enabling the binocular fusion of the first visual target signs s1 by having the examinee gaze at the left-side and right-side first visual target signs s1L, s1R. At this time, the left-side, first and second visual target signs s1L, s2L of the left visual targets OtL and the right-side, first and second visual target signs s1R, s2R of the right visual targets OtR are all displayed with the same brightness. This makes it possible to achieve the binocular fusion of the first visual target signs s1. Thus, the left subject eye EL is directed toward the left-side first visual target sign s1L with its visual line direction SL, while the right subject eye ER is directed toward the right-side first visual target sign s1R with its visual line direction SR.

Next, as shown in FIG. 10, the ophthalmologic apparatus 1 decreases only the brightness of the right-side first visual target sign s1R of the right visual targets OtR until the right subject eye ER has difficulty in visually recognizing the right-side first visual target sign s1R, while maintaining the brightness of the other three signs: namely, the left-side first and second visual target signs s1L, s2L, and the right-side second visual target sign s2R. When the right subject eye ER has difficulty in visually recognizing the right-side first visual target sign s1R, it is induced to gaze at the right-side second visual target sign s2R, whose brightness remains maintained, thereby shifting the right-side visual line direction SR toward the right-side second visual target sign s2R. At this time, since the brightness of the left-side first visual target sign s1L is maintained, the left subject eye EL maintains the left-side visual line direction SL toward the left-side first visual target sign s1L when the examinee gazes at the left-side first visual sign s1L. However, due to the difficulty of the right subject eye ER in recognizing the right-side first visual target sign s1R, the right-side second visual target sign s2R may serve as a binocular fusion stimulus. As a result, as shown in FIG. 11, the left subject eye EL may also be induced to gaze at the left-side second visual target sign s2L, thereby shifting the left-side visual line direction SL toward it. It is also possible to determine that the level of eyestrain is higher (i.e., the subject eyes are more fatigued) when the left-side visual line direction SL of the left subject eye EL is also shifted toward the left-side second visual target sign s2L (see FIG. 11), as compared with when only the right-side visual line direction SR of the right subject eye ER is shifted toward the right-side second visual target sign s2R (see FIG. 10).

**In** this way, the ophthalmologic apparatus 1 is configured such that the visual targets Ot each including the first visual target sign s1 and the second visual target sign s2, which has a different parallax from the first visual target sign s1. As a result, when the right subject eye ER has difficulty in visually recognizing the right-side first visual target sign s1R, the right subject eye ER can be induced to gaze at the right-side second visual target sign s2R, which has a different parallax from the first visual target sign s1. Thus, when the right subject eye ER has difficulty in visually recognizing the right-side first visual target sign s1R, the ophthalmologic apparatus 1 can shift the right-side visual line direction SR from the right-side first visual target sign s1R to the right-side second visual target sign s2R. Accordingly, the ophthalmologic apparatus 1 can properly detect the timing of the binocular fusion disruption of the right-side first visual target sign s1R.

In particular, the ophthalmologic apparatus 1 according to the first embodiment sets the convergence distance Lc, which is determined by the convergence angle θc of the first visual target signs s1, to be smaller than the visual target presentation distance Lp, and sets the convergence angle θc of the second visual target signs s2 to be smaller than that of the first visual target signs s1. Accordingly, when the right subject eye ER has difficulty in visually recognizing the right-side first visual target sign s1R, it is possible to more naturally guide the right-side visual line direction SR of the right subject eye ER toward the right-side second visual target sign s2R, because the right-side second visual target sign s2R is positioned in the divergence direction of the right subject eye ER and in a distance approaching the visual target presentation distance Lp. Thus, when the right subject eye ER has difficulty in visually recognizing the right-side first visual target sign s1R, the ophthalmologic apparatus 1 can more reliably guide the right-side visual line direction SR of the right subject eye ER toward the right-side second visual target sign s2R, thereby more reliably detecting the change in the right-side visual line direction SR.

**In** the ophthalmologic apparatus 1 according to the first embodiment, the controller 40 detects the focus points of the left and right subject eyes E (EL, ER), respectively, when detecting the visual line directions SL, SR of the left and right subject eyes E (EL, ER). The controller 40 can determine, from the detection results of the focus points, which locations (distances) the left and right subject eyes E (EL, ER) are gazing at. Accordingly, when determining whether the visual line directions SL, SR are changed, the controller 40 can appropriately determine, based on the detection results of the focus points, whether the right subject eye ER is gazing at the right-side first visual target sign s1R or the right-side second visual target sign s2R. This improves the accuracy of determining changes in the visual line directions SL, SR.

In the ophthalmologic apparatus 1 according to the first embodiment, when gradually increasing the brightness difference between the corresponding portions of the visual targets Ot, the controller 40 displays, on the display 31, the anterior ocular segment images E' of the left and right subject eyes EL, ER as ocular information obtained by using the anterior ocular segment observation systems 45, simultaneously with the increase in the brightness difference (contrast difference) in the left and right visual targets Ot. These anterior ocular segment images E' are ocular information used for detecting the visual line directions SL, SR. Thus, by observing the display 31, the examiner can monitor the movement of the left and right subject eyes E (EL, ER) while the brightness difference between the corresponding portions of the visual targets Ot is increasing, thereby determining whether the visual line directions SL, SR are changed. Based on this determination, the examiner can also determine the brightness difference at the moment of the binocular fusion disruption and estimate eyestrain. The examiner may also determine whether the visual line directions SL, SR are changed by directly observing the movement of the left and right subject eyes E (EL, ER) of the examinee.

In each visual target Ot of the ophthalmologic apparatus 1 according to the first embodiment, the second visual target sign s2 is the annular pattern surrounding the first visual target sign s1. This configuration allows the displacement between the center positions of the first visual target sign s1 and the second visual target sign s2, that is, the parallax difference between the first and second visual target signs s1, s2, to be minimized. Accordingly, when the right subject eye ER has difficulty in visually recognizing the right-side first visual target sign s1R, the right-side visual line direction SR of the right subject eye ER can be more reliably guided toward the right-side second visual target sign s2R.

The ophthalmologic apparatus 1 according to the first embodiment of the present invention can provide the following advantageous effects. The ophthalmologic apparatus 1 includes a visual target projection system 44 that is configured to present a left-eye visual target OtL to a left subject eye EL and a right-eye visual target OtR to a right subject eye ER. The left-eye and right-eye visual targets OtL, OtR have corresponding configurations and respectively include a corresponding portion s1L, s1R and a remaining portion s2L, s2R. The visual target projection system 44 is configured to increase a brightness difference between the corresponding portions s1L, s1R and maintain a brightness difference between the remaining portions s2L, s2R. Accordingly, when the binocular fusion of the corresponding portions of the left-eye and right-eye visual targets OtL, OtR is disrupted, the ophthalmologic apparatus 1 can guide the visual line directions SL, SR of the subject eye E, which has difficulty with visual recognition, toward the remaining portions of the left-eye and right-eye visual targets OtL, OtR. This enables the apparatus to reliably detect the change (ocular position displacement) of the visual line directions SL, SR. Thus, the ophthalmologic apparatus 1 enables the proper detection of the timing of the binocular fusion disruption and the appropriate estimation of eyestrain based on that timing.

In the ophthalmologic apparatus 1, the left-eye and right-eye visual targets OtL, OtR respectively includes two or more visual target signs s1L, s1R; s2L, s2R, each pair of the two or more visual target signs s1L, s1R; s2L, s2R of the left-eye and right-eye visual targets OtL, OtR being identical in shape. At least one of the two or more visual target signs s1L, s2L of the left-eye visual target OtL has different parallax from at least corresponding one of the two or more visual target signs s1R, s2R of the right-eye visual target OtR. Accordingly, the ophthalmologic apparatus 1 is configured to increase the brightness difference between a pair of visual target signs while maintaining the brightness differences between the remaining pairs of visual target signs. When one of the visual target signs becomes difficult to visually recognize due to reduced brightness, the ophthalmologic apparatus 1 can guide the visual line directions SL, SR toward the remaining visual target signs. This enables the ophthalmologic apparatus 1 to more reliably detect changes (ocular position displacement) in the visual line directions SL, SR.

In the ophthalmologic apparatus 1, a distance between the left subject eye EL and the left-eye visual target OtL and a distance between the right subject eye ER and the right-eye visual target OtR are each defined as a visual target presentation distance Lp. A point where a visual line of the left subject eye EL and a visual line of the right subject eye ER intersect is defined as a visual line intersection point Is. A line segment connecting the left subject eye EL and the visual line intersection point Is and a line segment connecting the right subject eye ER and the visual line intersection point Is defines a convergence angle Θc. The ophthalmologic apparatus 1 further includes a drive mechanism 23, which is a convergence adjustment mechanism, configured to adjust the convergence angle θc; an anterior ocular segment observation system 45, a refraction measurement projection system 46, and a refraction measurement light receiving system 47, which are an ocular information obtaining portion, configured to obtain ocular information of the left and right subject eyes EL, ER; and a controller 40 configured to control the visual target projection system 44, the drive mechanism 23, the anterior ocular segment observation system 45, the refraction measurement projection system 46, and the refraction measurement light receiving system 47. The controller 40 is configured to control the drive mechanism 23 to set the convergence angle θc to a desired value and the visual target projection system 44 to present the left-eye and right-eye visual targets OtL, OtR at the visual target presentation distance Lp; increase a brightness difference between one of the two or more visual target signs s1L, s2L of the left-eye visual target OtL and corresponding one of the two or more visual target signs s1R, s2R of the right-eye visual target OtR; maintain a brightness difference between remaining one of the two or more visual target signs s1L, s2L of the left-eye visual target OtL and corresponding remaining one of the two or more visual target signs s1R, s2R of the right-eye visual target OtR; detect visual line directions SL, SR of the left and right subject eyes EL, ER based on the ocular information obtained by the anterior ocular segment observation system 45, the refraction measurement projection system 46, and the refraction measurement light receiving system 47; determine a timing at which a binocular fusion of the left and right subject eyes EL, ER 9 is disrupted; and estimate an eyestrain level based on the timing at which the binocular fusion is disrupted. Accordingly, the ophthalmologic apparatus 1 can appropriately determine the timing at which the binocular fusion is disrupted and accurately estimate the level of eyestrain.

In the ophthalmologic apparatus 1, the left-eye visual target OtL includes a first visual target sign s1L and a second visual target sign s2L and the right-eye visual target OtR includes a first visual target sign s1R and a second visual target sign s2R. The first and second visual target signs s1L, s2L of the left-eye visual target OtL and the first and second visual target signs s1R, s2R of the right-eye visual target OtR are respectively identical in shape. The convergence angle θc for the second visual target signs s2L, s2R is smaller than the convergence angle θc for the first visual target signs s1L, s1R. The controller 40 increases the brightness difference between the first visual target signs s1L, s1R of the left-eye and right-eye visual targets OtL, OtR and maintains the brightness difference between the second visual target signs s2L, s2R of the left-eye and right-eye visual targets OtL, OtR. Accordingly, when the subject eye E has difficulty in visually recognizing the first visual target signs s1, the ophthalmologic apparatus 1 can more naturally guide the visual line directions SL, SR of the subject eye E toward the second visual target signs s2L, as the second visual target signs s2L are positioned in the divergence direction of the subject eye E.

The ophthalmologic apparatus 1 further includes a display to be viewed by at least an examiner. The controller 40 is configured to control the display to present an estimation result of the eyestrain level on the display. Accordingly, the ophthalmologic apparatus 1 can facilitate the understanding of the estimation result of the eyestrain level.

In the ophthalmologic apparatus 1, the controller 40 is configured to control the display 31 to present the ocular information obtained by the anterior ocular segment observation system 45, the refractive power measurement projection system 46, and the refractive power measurement light receiving system 47, which are the ocular information obtaining portion, when the visual target projection system 44, which is the visual target presenting mechanism, gradually increases the brightness difference between the first visual target signs s1L, s1R. Accordingly, by observing the display 31, the examiner can monitor the movement of the left and right subject eyes EL, ER as the brightness difference between the visual targets is increasing. This also enables the examiner to determine whether the visual line directions SL, SR are changed.

In the ophthalmologic apparatus 1, the controller 40 is configured to set a convergence distance Lc corresponding to the convergence angle θc to be shorter than the visual target presentation distance Lp. Accordingly, the ophthalmologic apparatus 1 can achieve the binocular fusion of the corresponding portions of the left-eye visual target OtL and the right-eye visual target OtR while the eyes are in a converged state, induce changes in the visual line directions SL, SR when the binocular fusion is disrupted, and facilitate the determination of the timing of the binocular fusion disruption.

In the ophthalmologic apparatus 1, the visual target projection system 44, which is the visual target presenting mechanism, includes a display 44a, which is a left-eye display, configured to present the left-eye visual target OtL and to adjust brightness of the left-eye visual target OtL, and a display 44a, which is a right-eye display 44a, configured to present the right-eye visual target OtR and to adjust brightness of the right-eye visual target OtR. Accordingly, the ophthalmologic apparatus 1 can finely adjust the brightness of the left-eye visual target OtL and the right-eye visual target OtR, and appropriately increase the brightness difference between them.

In the ophthalmologic apparatus 1, the controller 40 is configured to detect a focus point of the left subject eye EL and a focus point of the right subject eye ER based on the ocular information obtained by using the anterior ocular segment observation system 45, the refractive power measurement projection system 46, and the refractive power measurement light receiving system 47, which are the ocular information obtaining portion, when detecting the visual line directions of the left and right subject eyes EL, ER. Accordingly, the ophthalmologic apparatus 1 can appropriately determine, based on the detection result of the focus points of the left and right subject eyes E (EL, ER), whether the right subject eye ER is gazing at the right-side first visual target sign s1R or the right-side second visual target sign s2R. This improves the accuracy of determining changes in the visual line directions SL, SR.

Accordingly, the ophthalmologic apparatus 1 according to the present invention can facilitate the detection of the ocular position displacement when increasing the brightness difference between the visual targets Ot presented to the left and right subject eyes E under the binocular vision.

The ophthalmologic apparatus of the present invention has been described based on the first embodiment. However, the specific configurations of the ophthalmologic apparatus are not limited to those in the first embodiment. Design modifications, additions, and other changes are allowed as long as they do not depart from the gist of the inventions as defined in the claims.

For example, the first embodiment illustrates an application to the ophthalmologic apparatus 1 that observes, captures, and records the anterior ocular segment images, the ocular fundus images, and the ocular fundus tomographic images of the subject eyes, and provides them as the electronic images for diagnostic purposes. However, the ophthalmologic apparatus is not limited to the configuration of the first embodiment. The present invention can be applied to any ophthalmologic apparatus capable of increasing the brightness difference between the corresponding portions of the left-eye and right-eye visual targets OtL, OtR while maintaining the brightness difference between the remaining portions under the binocular vision, regardless of whether it is a subjective or objective ophthalmologic apparatus.

In the first embodiment, each of the first visual target signs s1 is formed as the asterisk design, and each of the second visual target signs s2 is formed as the annular design. However, the first and second visual target signs s1, s2 are not limited to the configurations of the first embodiment, as long as they are formed with different visual characteristics. In the first embodiment, the second visual target sign s2 surrounds the first visual target sign s1. However, as shown in FIG. 12, the second visual target sign s2 may be arranged next to the first visual target sign s1, and is not limited to the configuration of the first embodiment.

In the first embodiment, each of the first visual target signs s1 is presented at the center position of the display 44a, and each of the second visual target signs s2 is presented at a position that is displaced or off-centered outward from the center position of the display 44a (see FIG. 5). However, as long as the second visual target signs s2 are arranged so that its convergence angle θc is smaller than that of the first visual target signs s1, the arrangement is not limited to the first embodiment. Each of the first visual target signs s1 may be presented at a position that is displaced inward from the center position of the display, while each of the second visual target signs s2 may be presented at the center position. Alternatively, each of the first visual target signs s1 may be presented at a position that is displaced inward from the center position, while each of the second visual target signs s2 may be presented at a position that displaced outward from the center position.

In the first embodiment, each of the visual targets Ot, which includes the first and second visual target signs s1, s2, is displayed with the white signs on the black background. However, the visual targets Ot are not limited to those in the first embodiment. As long as visual targets Ot are visually recognizable and allow the first and second visual target signs s1, s2 to achieve respective binocular fusions of the first and second visual targets s1, s2, the colors of the background and signs may be set appropriately.

In the first embodiment, the ophthalmologic apparatus includes two visual target projection systems 44, which correspond to the left and right subject eyes E (EL, ER) respectively, as the visual target presenting mechanism. The visual target projection system 44 is capable of displaying any visual targets and includes a display 44a whose brightness can be adjusted. However, the visual target presenting mechanism is not limited to the configuration of the first embodiment as long as it has a function of increasing the brightness difference between either the first visual target signs s1 or the second visual target signs s2 while maintaining zero brightness difference between the other ones, the first visual target signs s1 or the second visual target signs s2. For example, the visual target presenting mechanism may include a liquid crystal shutter that is placed between the visual target and the left or right subject eye E (EL, ER) and that is configured to partially change its transmittance. Additionally, as long as the visual target presenting mechanism has the above-mentioned function, it does not necessarily have to be provided independently for each of the left and right subject eyes E (EL, ER).

The ophthalmologic apparatus 1 according to the first embodiment detects the focus points of the left and right subject eyes E (EL, ER) when determining whether the visual line directions SL, SR are changed. However, this detection is not necessarily required, as the focus points are detected to ensure the accuracy of determining changes in the visual line directions SL, SR.

In the ophthalmologic apparatus 1 according to the first embodiment, the display 31 of the examiner's control device 30 displays the eyestrain estimation results of the left and right subject eyes E (EL, ER) and the anterior ocular segment images E' of the left and right subject eyes E (EL, ER) when gradually increasing the brightness difference between the visual targets. However, as long as the examiner can visually recognize the information, other display devices such as a monitor installed on the optometric table 12 or a display provided on the measurement unit 20 may also be used.

The ophthalmologic apparatus 1 according to the first embodiment stops the increase in the brightness difference between the corresponding portions of the visual targets Ot when the change in the visual line directions SL, SR begins, to determine the brightness difference at the moment of the binocular fusion disruption. However, the controller 40 may continue detecting the change in the visual line directions SL, SR until the brightness of the corresponding portions of one of the visual targets Ot reaches zero, that is, until the corresponding portions turn to have the same color as that of the background. Additionally, the visual line directions SL, SR may be continuously acquired by adjusting the brightness difference in fine increments or by continuously varying the brightness difference. A graph representing the relationship between the brightness difference and the change in the visual line directions SL, SR may be used to determine the brightness difference at the moment of the binocular fusion disruption based on a steep slope in the graph. Alternatively, the focus point of the subject eye E (EL, ER) may be continuously detected, and a period with significant fluctuation in the focus point may be identified as the brightness difference at the moment of the binocular fusion disruption. Furthermore, the brightness difference at the moment of the binocular fusion disruption may be determined by combining the change in the visual line directions SL, SR with the variation in the focus point.

In the ophthalmologic apparatus 1 according to the first embodiment, each of the visual targets Ot includes the first and second visual target signs s1, s2. However, the visual target is not limited to the configuration of the first embodiment. Each of the visual targets may include three or more visual target signs as long as it includes multiple visual target signs with different parallaxes. For example, if the visual target includes a third visual target sign in addition to the first and second visual target signs s1, s2, the third visual target sign may be placed in an inwardly shifted position relative to the first visual target sign s1. This arrangement allows the visual line direction to naturally shift toward the third visual target sign, even when the visual line tends to move in the convergence direction due to difficulty in visually recognizing the first visual target sign s1. Accordingly, this configuration enables reliable detection of the movement of the visual line directions SL, SR from the first visual target sign s1.

In the ophthalmologic apparatus 1 according to the first embodiment, each of the visual targets Ot includes the first and second visual target signs s1, s2. However, the visual target Ot may use the edge of the display 44a as the second visual target signs s2, meaning that only the first visual target sign s1 is presented on the display 44a. In this case, the first visual target sign s1 is presented at a position that is displaced inward from the center of the display 44a (right side if it is the left-side first visual target sign s1L and left side if it is the right-side first visual target sign s1R). The brightness difference is determined as the brightness difference at the moment of the binocular fusion disruption when the visual line directions SL, SR shift due to increasing the brightness difference between the left-side and right-side first visual target signs s1L, s1R under the binocular fusion of the first visual target signs s1 or when the examinee reports seeing double images of the first visual target sign s1. When the examinee finds it difficult to visually recognize the first visual target signs s1, the visual line directions SL, SR of the subject eye E may be guided toward the edge of the display 44a, which serves as the second visual target signs s2. This configuration provides effects similar to those of the first embodiment.
(1) An ophthalmologic apparatus comprising:
   a visual target presenting mechanism that is configured to present a left-eye visual target to a left subject eye and a right-eye visual target to a right subject eye,
   wherein the left-eye and right-eye visual targets have corresponding configurations and respectively comprise a corresponding portion and a remaining portion, and
   wherein the visual target presenting mechanism is configured to increase a brightness difference between the corresponding portions and maintain a brightness difference between the remaining portions.
(2) The ophthalmologic apparatus according to the above (1),
   wherein the left-eye and right-eye visual targets respectively comprise two or more visual target signs, each pair of the two or more visual target signs of the left-eye and right-eye visual targets being identical in shape, and
   wherein at least one of the two or more visual target signs of the left-eye visual target has different parallax from at least corresponding one of the two or more visual target signs of the right-eye visual target.
(3) The ophthalmologic apparatus according to the above (2),
   wherein a distance between the left subject eye and the left-eye visual target and a distance between the right subject eye and the right-eye visual target are each defined as a visual target presentation distance,
   wherein a point where a visual line of the left subject eye and a visual line of the right subject eye intersect is defined as a visual line intersection point,
   wherein a line segment connecting the left subject eye and the visual line intersection point and a line segment connecting the right subject eye and the visual line intersection point defines a convergence angle,
   wherein the ophthalmologic apparatus further comprises:
      a convergence adjustment mechanism that is configured to adjust the convergence angle;
      an ocular information obtaining portion that is configured to obtain ocular information of the left and right subject eyes; and
      a controller that is configured to control the visual target presenting mechanism, the convergence adjustment mechanism, and the ocular information obtaining portion, and
      wherein the controller is configured to
      control the convergence adjustment mechanism to set the convergence angle to a desired value and the visual target presenting mechanism to present the left-eye and right-eye visual targets at the visual target presentation distance,
      increase a brightness difference between one of the two or more visual target signs of the left-eye visual target and corresponding one of the two or more visual target signs of the right-eye visual target,
      maintain a brightness difference between remaining one of the two or more visual target signs of the left-eye visual target and corresponding remaining one of the two or more visual target signs of the right-eye visual target,
      detect visual line directions of the left and right subject eyes based on the ocular information obtained by the ocular information obtaining portion,
      determine a timing at which a binocular fusion of the left and right subject eyes is disrupted, and
      estimate an eyestrain level based on the timing at which the binocular fusion is disrupted.
(4) The ophthalmologic apparatus according to the above (3),
   wherein the left-eye visual target comprises a first visual target sign and a second visual target sign and the right-eye visual target comprises a first visual target sign and a second visual target sign,
   wherein the first and second visual target signs of the left-eye visual target and the first and second visual target signs of the right-eye visual target are respectively identical in shape,
   wherein the convergence angle for the second visual target signs is smaller than the convergence angle for the first visual target signs, and
   wherein the controller increases the brightness difference between the first visual target signs of the left-eye and right-eye visual targets and maintains the brightness difference between the second visual target signs of the left-eye and right-eye visual targets.
(5) The ophthalmologic apparatus according to the above (3) or (4), further comprising a display to be viewed by at least an examiner, and
   wherein the controller is configured to control the display to present an estimation result of the eyestrain level on the display.
(6) The ophthalmologic apparatus according to the above (5), wherein the controller is configured to control the display to present the ocular information obtained by the ocular information obtaining portion as the visual target presenting mechanism gradually increases the brightness difference between the first visual target signs.
(7) The ophthalmologic apparatus according to any one of the above (3) to (6), wherein the controller is configured to set a convergence distance corresponding to the convergence angle to be shorter than the visual target presentation distance.
(8) The ophthalmologic apparatus according to any one of the above (3) to (7),
   wherein the visual target presenting mechanism comprises
   a left-eye display that is configured to present the left-eye visual target and to adjust brightness of the left-eye visual target, and
   a right-eye display that is configured to present the right-eye visual target and to adjust brightness of the right-eye visual target.
(9) The ophthalmologic apparatus according to any one of the above (3) to (8), wherein the controller is configured to detect a focus point of the left subject eye and a focus point of the right subject eye based on the ocular information obtained by the ocular information obtaining portion when detecting the visual line directions of the left and right subject eyes.

### List of Reference Signs

1 ophthalmologic apparatus
23 drive mechanism (an example of convergence adjustment mechanism)
31 display
40 controller
44 visual target projection system (an example of visual target presenting mechanism)
44a display (examples of left-eye display and right-eye display)
45 anterior ocular segment observation system (an example of ocular information obtaining portion)
46 refraction measurement projection system (an example of ocular information obtaining portion)
47 refraction measurement light receiving system (an example of ocular information obtaining portion)
EL left subject eye
ER right subject eye
Is visual line intersection point
Lp visual target presentation distance
OtL left-eye visual target
OtR right-eye visual target
s1 first visual target sign
s2 second visual target sign
θc convergence angle

## Claims

1. An ophthalmologic apparatus (1) comprising:
a visual target presenting mechanism (44) that is configured to present a left-eye visual target (OtL) to a left subject eye (EL) and a right-eye visual target (OtR) to a right subject eye (ER),
wherein the left-eye and right-eye visual targets (OtL, OtR) have corresponding configurations and respectively comprise a corresponding portion (s1L, s1R) and a remaining portion (s2L, s2R), and
wherein the visual target presenting mechanism (44) is configured to increase a brightness difference between the corresponding portions (s1L, s1R) and maintain a brightness difference between the remaining portions (s2L, s2R).

2. The ophthalmologic apparatus (1) according to claim 1,
wherein the left-eye and right-eye visual targets (OtL, OtR) respectively comprise two or more visual target signs (s1L, s1R; s2L, s2R), each pair of the two or more visual target signs (s1L, s1R; s2L, s2R) of the left-eye and right-eye visual targets (OtL, OtR) being identical in shape, and
wherein at least one of the two or more visual target signs (s1L, s2L) of the left-eye visual target (OtL) has different parallax from at least corresponding one of the two or more visual target signs (s1R, s2R) of the right-eye visual target (OtR).

3. The ophthalmologic apparatus (1) according to claim 2,
wherein a distance between the left subject eye (EL) and the left-eye visual target (OtL) and a distance between the right subject eye (ER) and the right-eye visual target (OtR) are each defined as a visual target presentation distance (Lp),
wherein a point where a visual line of the left subject eye (EL) and a visual line of the right subject eye (ER) intersect is defined as a visual line intersection point (Is),
wherein a line segment connecting the left subject eye (EL) and the visual line intersection point (Is) and a line segment connecting the right subject eye (ER) and the visual line intersection point (Is) defines a convergence angle (θc),
wherein the ophthalmologic apparatus (1) further comprises:
a convergence adjustment mechanism (23) that is configured to adjust the convergence angle (θc);
an ocular information obtaining portion (45, 46, 47) that is configured to obtain ocular information of the left and right subject eyes (EL, ER); and
a controller (40) that is configured to control the visual target presenting mechanism (44), the convergence adjustment mechanism (23), and the ocular information obtaining portion (45, 46, 47), and
wherein the controller (40) is configured to
control the convergence adjustment mechanism (23) to set the convergence angle (θc) to a desired value and the visual target presenting mechanism (44) to present the left-eye and right-eye visual targets (OtL, OtR) at the visual target presentation distance (Lp),
increase a brightness difference between one of the two or more visual target signs (s1L, s2L) of the left-eye visual target (OtL) and corresponding one of the two or more visual target signs (s1R, s2R) of the right-eye visual target (OtR),
maintain a brightness difference between remaining one of the two or more visual target signs (s1L, s2L) of the left-eye visual target (OtL) and corresponding remaining one of the two or more visual target signs (s1R, s2R) of the right-eye visual target (OtR),
detect visual line directions (SL, SR) of the left and right subject eyes (EL, ER) based on the ocular information obtained by the ocular information obtaining portion (45, 46, 47),
determine a timing at which binocular fusion of the left and right subject eyes (EL, ER) is disrupted, and
estimate an eyestrain level based on the timing at which the binocular fusion is disrupted.

4. The ophthalmologic apparatus (1) according to claim 3,
wherein the left-eye visual target (OtL) comprises a first visual target sign (s1L) and a second visual target sign (s2L) and the right-eye visual target (OtR) comprises a first visual target sign (s1R) and a second visual target sign (s2R),
wherein the first and second visual target signs (s1L, s2L) of the left-eye visual target (OtL) and the first and second visual target signs (s1R, s2R) of the right-eye visual target (OtR) are respectively identical in shape,
wherein the convergence angle (θc) for the second visual target signs (s2L, s2R) is smaller than the convergence angle (θc) for the first visual target signs (s1L, s1R), and
wherein the controller (40) increases the brightness difference between the first visual target signs (s1L, s1R) of the left-eye and right-eye visual targets (OtL, OtR) and maintains the brightness difference between the second visual target signs (s2L, s2R) of the left-eye and right-eye visual targets (OtL, OtR).

5. The ophthalmologic apparatus (1) according to claim 3 or 4, further comprising a display to be viewed by at least an examiner, and
wherein the controller (40) is configured to control the display to present an estimation result of the eyestrain level on the display.

6. The ophthalmologic apparatus (1) according to claim 5, wherein the controller (40) is configured to control the display to present the ocular information obtained by the ocular information obtaining portion (45, 46, 47) as the visual target presenting mechanism (44) gradually increases the brightness difference between the first visual target signs (s1L, s1R).

7. The ophthalmologic apparatus (1) according to any one of claims 3 to 6, wherein the controller (40) is configured to set a convergence distance (Lc) corresponding to the convergence angle (θc) to be shorter than the visual target presentation distance (Lp).

8. The ophthalmologic apparatus (1) according to any one of claims 3 to 7,
wherein the visual target presenting mechanism (44) comprises
a left-eye display (44a) that is configured to present the left-eye visual target (OtL) and to adjust brightness of the left-eye visual target (OtL), and
a right-eye display (44a) that is configured to present the right-eye visual target (OtR) and to adjust brightness of the right-eye visual target (OtR).

9. The ophthalmologic apparatus (1) according to any one of claims 3 to 8, wherein the controller (40) is configured to detect a focus point of the left subject eye (EL) and a focus point of the right subject eye (ER) based on the ocular information obtained by the ocular information obtaining portion (45, 46, 47) when detecting the visual line directions of the left and right subject eyes (EL, ER).
